# EUROPEAN PATENT SPECIFICATION

(11) **EP 2 806 814 B1**
(45) Date of publication and mention of the grant of the patent: **27.01.2021**
(21) Application number: 12866594.0
(22) Date of filing: 18.12.2012
(51) Int. Cl.: A61B 18/02, A61B 90/00, A61B 18/00

(54) **CRYOGENIC MEDICAL SYSTEM WITH STABILIZER**
KRYOGENES MEDIZINISCHES SYSTEM MIT EINEM STABILISATOR
SYSTÈME MÉDICAL CRYOGÉNIQUE AVEC STABILISATEUR

(30) Priority: 27.01.2012 US 201261591338 P; 04.04.2012 US 201213439039
(43) Date of publication of application: 03.12.2014
(73) Proprietor: Medtronic Cryocath LP, Toronto, Ontario M5L 1B9 (CA)
(72) Inventor: MONGER, Eric, Beloeil, Québec J3G 2H7 (CA)
(74) Representative: FRKelly
(86) International application number: PCT/CA2012/001159
(87) International publication number: WO 2013/110154

(56) References cited:
- WO-A1-03/034003
- CA-A1- 2 547 953
- US-A- 1 536 614
- US-A- 2 902 271
- US-A- 4 541 276
- US-A- 5 417 073
- US-A- 6 119 475
- US-A1- 2009 143 923

## Description

### FIELD OF THE INVENTION

The present invention relates to a cryogenic medical device.

### BACKGROUND OF THE INVENTION

Cryogenic medical devices and systems are often employed for medical procedures, including those involving mapping, ablation, dilation, and the like. For example, a thermal diagnostic or treatment procedure may involve exchanging thermal energy with a targeted tissue region. Cryogenic medical procedures may use various materials to produce extremely low temperatures. Such materials may include cryogenic fluids such as liquefied gases, for example liquid nitrogen or liquid helium. Cryogenic fluids present challenges in safely and effectively storing, transporting and using them. Accordingly, the cryogenic fluids may be contained in fixed-volume tanks of certain sizes, which may be relatively heavy and bulky.

An example of a thermal mechanism for diagnosis and treatment is a cryogenic device that uses thermal energy transfer from thermodynamic changes occurring during the flow of a cryogen through the device to cause a net transfer of heat from the target tissue to a portion of the device. The cryogen may flow from a tank or reservoir to the treatment portion of a medical device through one or more conduits, tubular structures, regulators, valves, and other fluid flow components.

As the cryogen is used during successive medical treatments, the amount of cryogenic fluid remaining in a tank or reservoir will reduce, which may be monitored by weighing the tank over time. The resulting weight measurements may be compared to known values for the weight of the tank in various conditions ranging from empty to full capacity. Specifically, the weight of the tank and its contents may be measured with various sensors, including a scale or other load sensor. However, the numeric values of measurements from the load sensor may be affected by the position and orientation of the tank relative to the load sensor. In other words, the load sensor will produce the most accurate results when the tank fully engages the load sensor, and also has the proper orientation or alignment relative to the load sensor.

As an example, most current configurations include a console with an injection panel, cryogen tank connected to the injection panel, and a load sensor. If the tank does not fully engage the load sensor or if the tank is tilted or otherwise misoriented, the amount of stress on the injection hose connecting the tank to the injection panel may be altered, thereby changing the pressure of the tank on the load sensor. As a result, the reading of the cryogenic fluid level may inaccurately reflect a change of between approximately 4.536 g and approximately 1.814 kg (approximately .01 and approximately 4.0 lbs), and can have a significant effect on time management for a particular medical procedure. Such inaccurate readings may be prevented by a reservoir stabilization system that allows the free vertical movement of the tank, thus avoiding or reducing stress on the injection hose.

Accordingly, it is desirable to provide systems and methods of use thereof that provide more accurate measurement of the weight of the tank and its contents. It is also desirable to avoid damage to the fluid flow components by resisting tipping or displacement of the tank of cryogenic fluid.

United States patent specification no. US -A - 4 541 276 A1 discloses that the fluid contents of a closed container, particularly a closed cryogen container, are gauged by lifting the container through a lever arm one end of which acts upon a pressure transducer coupled to indicating means while the other end of the arm is spring biased to suppress the empty or tare weight of the container.

United States patent specification no. US -A - 2 902 271 A1 discloses weighing devices and has particular reference to a device for indicating a constant weight of fuel tanks and their contents, particularly fuel tanks of the recognized bottled gas.

United States patent specification no. US -A - 6 119 475 A1 discloses a lift assembly which is coupled to a refrigerant service cart to raise and lock the supply tank of refrigerant off of a weight sensor, locking the tank in a raised position for transportation of the portable servicing cart. In a preferred embodiment, a fixed arm extends from the portable cart and is positioned at a location above the refrigerant tank. A movable connecting link extends from the arm and is coupled to the tank and is controlled by an over-centre toggle with a control handle for raising and lowering the tank coupled to the link between a use position resting on the weight sensor and a transporting position engaging the fixed arm and locked thereto by the over-centre toggle control.

Documents US1536614 A, CA2547953 A1 and WO03034003 A1 disclose further relevant background art.

### SUMMARY OF THE INVENTION

According to an aspect of the present invention, there is provided a medical device as specified in claim 1. According to another aspect of the present invention, there is provided a medical device as specified in any of claims 2 - 8.

The present disclosure advantageously provides systems and methods of use for cryogenic medical treatment having a stabilizer for a reservoir of cryogenic fluid, allowing the reservoir a limited range of motion. In particular, a medical device is provided, including a load sensor, a reservoir containing cryogenic fluid and engaging the load sensor, and a stabilizer restraining the reservoir within a limited range of motion relative to the load sensor.

The medical device stabilizer allows vertical movement of the reservoir relative to the load sensor. The medical device stabilizer is operable to resist tipping of the reservoir, and is operable to substantially maintain a predetermined orientation of the reservoir relative to the load sensor. Further, the medical device stabilizer is directly or indirectly coupled with the reservoir.

The medical device has a control unit and a conduit providing fluid communication between the reservoir and the control unit, such that the stabilizer resists tipping of the reservoir and disruption of the fluid communication. Further, the medical device is operable to measure the weight of the reservoir and the cryogenic fluid.

A medical device is provided, including a control unit, a reservoir containing cryogenic fluid, a load sensor operable to weigh the reservoir and cryogenic fluid, a conduit providing fluid communication between the reservoir and the control unit, and a coupling limiting movement of the conduit relative to the control unit within a range of positions. The medical device coupling has a bracket and a guide member, each being coupled to the conduit or the control unit.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is an illustration of an example of a medical system constructed in accordance with the principles of the present disclosure;
FIG. 2 is an illustration of another example of a medical system constructed in accordance with the principles of the present disclosure;
FIG. 3 is an illustration of a medical system constructed in accordance with the principles of the present invention;
FIG. 4 is an illustration of still another example of a medical system constructed in accordance with the principles of the present disclosure
FIG. 5 is a partial perspective view of a first stabilizer for a medical system constructed in accordance with the principles of the present disclosure;
FIG. 6 is a partial perspective view of a second stabilizer for a medical system constructed in accordance with the principles of the present disclosure;
FIG. 7 is an illustration of a third stabilizer for a medical system in accordance with the principles of the present disclosure;
FIG. 8 is an illustration of a fourth stabilizer for a medical system in accordance with the principles of the present disclosure;
FIG. 9 is an illustration of a fifth stabilizer for a medical system in accordance with the principles of the present disclosure;
FIG. 10 is an illustration of a sixth stabilizer for a medical system in accordance with the principles of the present disclosure;
FIG. 11 is an illustration of a seventh stabilizer for a medical system in accordance with the principles of the present disclosure;
FIG. 12 is an illustration of an eighth stabilizer for a medical system in accordance with the principles of the present disclosure; and
FIG. 13 is an illustration of medical system in accordance with the principles of the present disclosure.

### DETAILED DESCRIPTION OF THE INVENTION

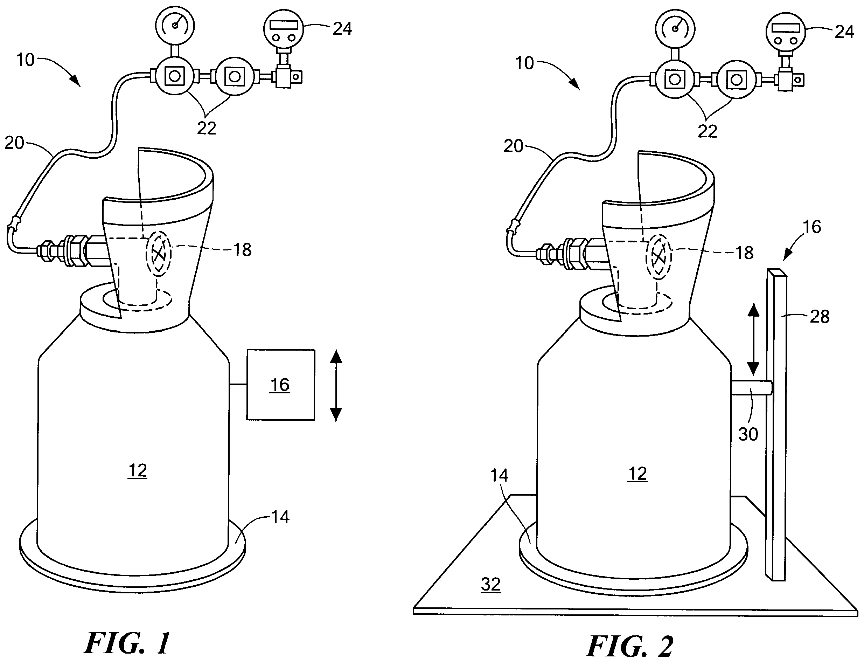

The present disclosure advantageously provides systems and methods of use thereof for cryogenic medical treatment, having a stabilizer for a reservoir of cryogenic fluid. Referring now to the drawing figures, in which like reference designations refer to like elements, an embodiment of a medical system constructed in accordance with principles of the present disclosure is shown and generally designated as "10." Referring now to FIG. 1, the medical system 10 generally includes a reservoir 12 adapted to contain cryogens such as cryogenic fluids. To monitor the amount of cryogenic fluid inside the reservoir 12, the reservoir 12 may sit upon or otherwise engage a load sensor 14, which may be operable to measure the current combined weight of the reservoir 12 and whatever amount of cryogenic fluid is inside the reservoir 12 at the time. The medical system 10 may also have a stabilizer 16 that allows the reservoir some limited range of motion, and restricts movement of the reservoir 12 beyond the desired range of motion.

This limited range of motion may serve to enhance accuracy of the measurements of the load sensor 14 by allowing the reservoir 12 to sit properly and to correctly engage the load sensor 14, while also adapting to and correcting situations in which the reservoir 12 becomes misplaced or misaligned (either momentary or lasting). For example, the system 10 and stabilizer 16 may allow the reservoir 12 a certain amount of motion in one or more vertical or horizontal directions, or rotation about any selected axis, or any combination thereof. Vertical motion of the reservoir 12 may allow for accurate and unhindered operation of the load sensor 14, while horizontal motion of the reservoir 12 may allow for correct placement on and engagement of the load sensor 14. A limited range of rotation of the reservoir 12 about an axis, such as a horizontal axis, may accommodate forces tending to tip the reservoir 12 and resist the reservoir 12 from tipping over or otherwise falling out of alignment with the load sensor 14. The stabilizer 16 may thus resist tipping or other undesirable movement by the reservoir 12, and/or substantially maintain a predetermined orientation of the reservoir 12 relative to the load sensor 14. However, the stabilizer 16 should also provide for easy removal and insertion of the reservoir 12, such as when the reservoir 12 becomes empty and must be substitute by a new reservoir 12.

The load sensor 14 may be of any suitable type, for example one or more load cells or transducers in various configurations, which may include one or more strain gauges or other sensors of tension, compression, force, pressure, torque, and the like. An example load sensor may include multiple strain gauges arranged in different orientations that are deformed by mechanical forces on the load sensor, which then measures that deformation and produces at least one electrical signal. Based on these signals, the weight of the reservoir 12 and cryogens contained therein may be calculated. Other types of load cells for use in a load sensor may include hydraulic or hydrostatic load cells, or piezoelectric load cells. The load sensor 14 may be operated manually or automatically obtain weight measurements on a continuous, continual, repeating or periodic basis.

Continuing to refer to FIG. 1, the reservoir 12 may be coupled to or through one or more fluid handling components such as a valve 18, a conduit 20, a regulator 22, and a control unit 24. The valve 18 may prevent, allow, regulate, direct, or otherwise control flow of cryogenics fluid in the reservoir 12 and other fluid flow components. The valve 18 may be, for example, binary (on/off) or graduated, and may be operated manually or automatically.

Continuing to refer to FIG. 1, the conduit 20 may be any tubular structure capable of maintaining pressurized cryogens and the associated fluid flow, and may be made of any selected materials having the desired characteristics such as pressure integrity. For example, the conduit 20 may be made of metals or polymers, or a combination thereof. All of the conduit 20 or a portion thereof may have a selected flexibility or inflexibility, and different portions may have different flexibilities. Some or the entire conduit 20 may have a fixed length, or it may allow some degree of longitudinal stretching. Another advantage of the stabilizer 16 is that the resulting limited range of motion for the reservoir 12 resists the reservoir 12 (which may be relatively heavy) from tipping over and possibly damaging or disconnecting the conduit 20, enhancing safety of the system 10.

Continuing to refer to FIG. 1, the regulator 22 may be operable to manipulate, modulate, and otherwise regulate the flow of cryogens during operation of the system 10 for medical treatment. The control unit 24 may be operable to automatically operate the other components of the system 10, and which may include activation and deactivation, monitoring and feedback evaluation. The control unit 24 may include various dials, levers, knobs, gauges, buttons, screens, and other displays and/or user input devices that allow for the manipulation of certain system 10 components.

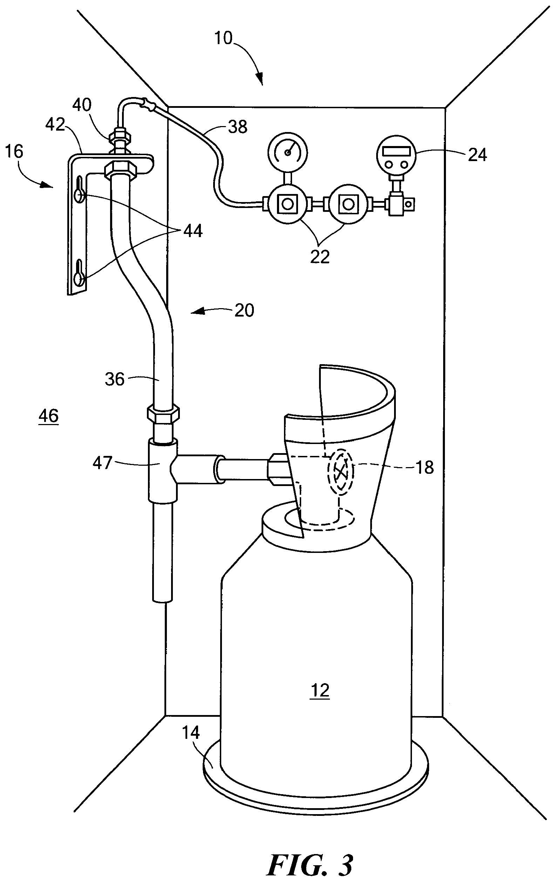

Referring now to FIG. 2, the medical system 10 may have a particular example of a stabilizer 16 that includes a support 28 and a coupling 30. The support 28 may have the characteristics of a stable structure or foundation connected to the load sensor 14 directly or indirectly through a base 32, or another intermediary structure, equipment, or physical ground reference such as a floor. The support may take various forms, for example, a bar or rod, frame, lattice, scaffold, housing, box, or container. The coupling 30 may have any suitable shape, size, or arrangement that allows for the desired range of motion of the reservoir 12 relative to the load sensor 14 (as shown in FIGS. 3-12). The coupling 30 may have a mechanism allowing relative movement of the reservoir 12 and the support 28, and yet limit such movement to the desired ranges of positions and/or orientation. For example, the coupling 30 may include a guide member (such as shown in FIG. 3) coupled to the reservoir 12 and a support 28 coupled to the load sensor 14, the guide member being movable relative to the support 28. Accordingly, the stabilizer 26 may be directly or indirectly coupled with the reservoir 12.

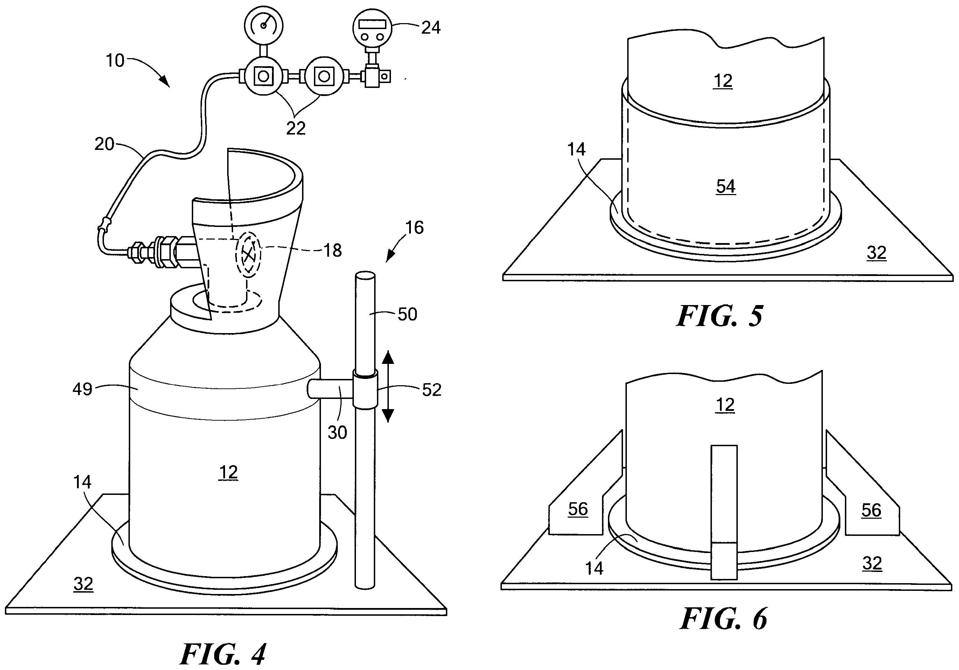

Referring now to FIG. 3, according to the invention, the system 10 includes a reservoir 12 containing cryogenic fluid and sitting on a load sensor 14 operable to weigh the reservoir and cryogenic fluid, a valve 18, one or more regulators 22, and a control unit 24. In particular, the system 10 also has a conduit 20 providing fluid communication between the reservoir 12 and regulator 22 and control unit 24. The conduit 20 is a tubular assembly and has a compound structure with a first tube 36 and a second tube 38. The conduit 20 acts as a stabilizer 16, or may be used in conjunction with other stabilizers 16 as shown in FIGS. 4-11.

The conduit 20 may be any tubular structure suitable for maintaining fluid flow under pressure and having other desirable characteristics, and its components may be made of various materials such as metals and polymers. In the particular example shown in FIG. 3, the first tube 36 has a substantially constant length, and the second tube 38 is more flexible than the first tube 36. The junction of the first tube 36 and the second tube 38 is attached to, affixed to or otherwise engaging a coupling 40 which is, in turn, connected to a structure that is directly or indirectly connected to the load sensor 14. For example, the system 10 shown in FIG. 3 is located within a larger outer container or console as is commonly used in association with ablation procedures, and the coupling 40 is connected to or mounted on one or more structures affixed to one of the inner walls of the console. As shown in FIG. 3, the coupling 40 includes a bracket 42 connected to the conduit 20 and one or more guide members 44 connected directly or indirectly to the load sensor 14 or a base structure 46 (such as the console inner wall, as shown in FIG. 3), thus limiting movement of a portion of the conduit 20 at a junction between the first tube 36 and the second tube 38 within a range of positions relative to the control unit 24. Alternatively, the bracket 42 may be connected to the load sensor 14 or base structure 46, and the guide members 44 in turn connected to the conduit 20. The first tube 36 is further coupled to an inflexible connector element 47, as shown in FIG. 3, which facilitates connection of the conduit 20 to and/or disconnection from the reservoir 12. The connector element 47 may include a handle and/or third tube that may be connected to the reservoir 12. The coupling 40 operates to allow the reservoir 12 a limited range of a free range of motion in vertical directions, and to substantially limit horizontal or tipping motion of the reservoir 12. Accordingly, this limit to the motion and/or rotation of the reservoir 12 tends to avoid damage to the conduit 20 and possible disruption of fluid communication through the conduit 20.

Referring now to FIG. 4, the stabilizer 16 may include a rail 50 and a follower 52. The rail 50 may be a vertical track or rod as shown in FIG. 4, or may have any other shape, cross-section, or curvature. The follower 52 may fully or partially surround the rail 50, and fits so as to allow free vertical movement of the follower 52 along the rail 50. The follower 52 may be in direct or indirect contact with the reservoir 12, so as to allow for corresponding vertical movement of both the reservoir 12 and follower 52. For example, as shown in FIG. 4, the follower 52 may be coupled to a band, strap, hoop, or other device 49 in contact with the reservoir 12.

Referring now to FIGS. 5 and 6, a stabilizer 16 may take the form of a wall, buttress, support, or any other element that has a portion positioned a vertical distance from the load sensor, that may contact or define a gap between that portion of the stabilizer and the reservoir 12. In the particular example of FIG. 5, the stabilizer 16 may include a retaining wall 54. Retaining wall 54 may have a curved or closed loop shape as shown in FIG. 5, or may have straight, multi-sided, polygonal, segmented, or other shape providing the desired effects of retaining the reservoir 12 substantially within a desired range of positions and orientation relative to the load sensor 14, while resisting undesirable tipping, rotational or other movement. In the particular example of FIG. 6, the stabilizer 16 may include a series of supports 56. Supports 56 may have a straight, angled, or curved shape, or may have the shape of an architectural buttress, or other shapes providing the desired effects of retaining the reservoir 12 substantially within a desired range of positions and orientation relative to the load sensor 14, while resisting undesirable tipping, rotational or other movement. The stabilizers shown in FIGS. 5 and 6 may be used in addition to the conduit 20 assembly.

In another example, the stabilizer 16 or retaining elements may be movable or adjustable between a first position substantially retaining the reservoir in an operating position, as shown in FIGS. 5 and 6, and a second position facilitating placement and removal of the reservoir in the operating position. For example, the supports 56 of FIG. 6 may be resiliently deformable (for example, made of rubber or a strong foam capable of supporting the reservoir 12) to facilitate insertion or removal of the reservoir 12. As an alternative example, the supports 56 of FIG. 6 may be rotatably coupled to a base 32 or may swivel to an open position (not shown).

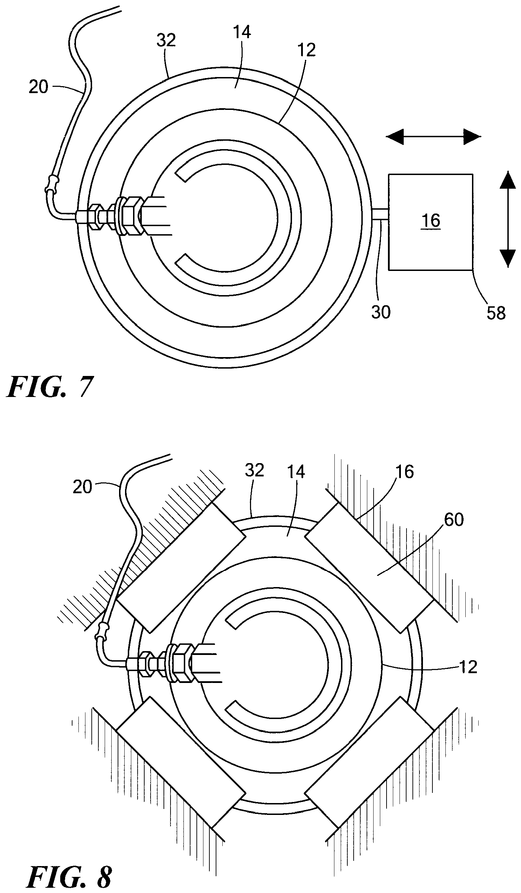

Referring now to FIGS. 7-12, the stabilizer 16 may have various features, designs, components, and configurations. FIGS. 7-12 are shown from an elevated perspective, above the nozzle portion of the reservoir 12 (that is, above the top of the reservoir 12 when the reservoir 12 is vertically situated for normal use). In FIG. 7, for example, a stabilizer 16 may provide for a limited range of motion of the reservoir 12 in orthogonal directions. A possible example may include one or more gyroscope devices 58, which in operation tend to maintain rotational orientation or alignment. Accordingly, one or more gyroscopes 58 may be directly or indirectly coupled to the reservoir 12 (for example, the gyroscopes 58 may be connected to couplers 30, as shown in FIG. 7), and each gyroscope 58 may be arranged so as to limit motion or rotation of the reservoir 12 along a selected axis.

With reference to FIG. 8, the stabilizer 16 may include retaining elements such as an arrangement of one or more guides or resilient bumpers 60. The bumpers 60 may be positioned in various directions around the reservoir 12, and may be arranged to continuously surround the reservoir 12 or in discrete positions. The bumpers 60 may be in contact with the reservoir 12 or may define a gap between the bumpers 60 and the reservoir 12.

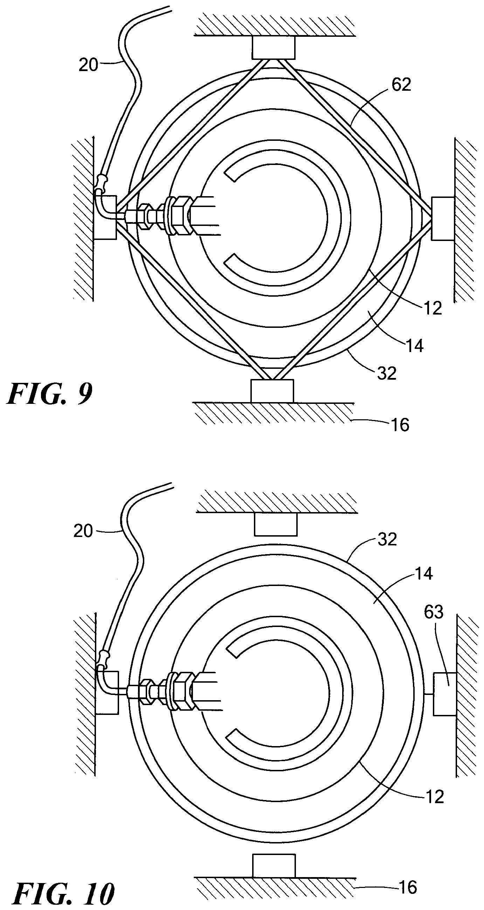

With reference to FIG. 9, the stabilizer 16 may impose resilient forces tending to retain the reservoir in the desired position. A stabilizer 16 may include retaining elements such as one or more resilient members, for example elastic bands 62 connected to one or more parts of the stabilizer 16. Accordingly, the freedom of horizontal movement of the reservoir 12 would be a function of the elasticity of the elastic bands 62.

With reference to FIG. 10, the stabilizer 16 may utilize one or more magnetic field generators 63, arranged to impose magnetic forces and retain the reservoir 12 in the desired position and orientation. For example, the magnetic field generators 63 may be positioned in various directions around the reservoir 12, and may be arranged to continuously surround the reservoir 12 or in discrete positions (as, for example, the bumpers 60 of FIG. 8). Further, the magnetic field generators 63 may be in contact with the reservoir 12 (for example, when the magnetic field generators 63 exert an attractive force on the reservoir 12) or may define a gap between the bumpers 60 and the reservoir 12 (for example, when the magnetic field generators 63 exert a repellent force on the reservoir 12).

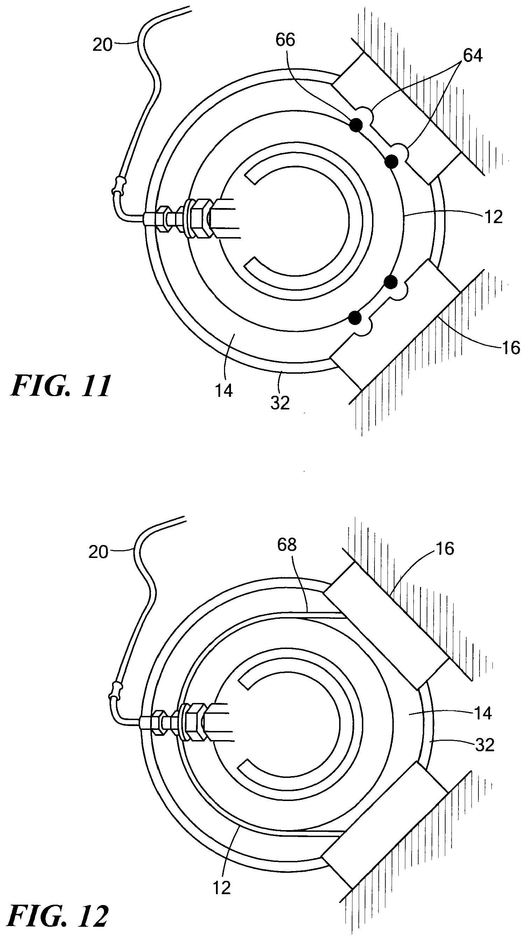

With reference to FIG. 11, the stabilizer 16 may be one or more tracks 64 and followers 66, in which each track 64 or follower 66 partially surrounds the other. The tracks 64 may be arranged vertically or in other directions, and may be straight, curvilinear, or follow another desired path. The followers 66 may be pegs, rods, or other elements capable of fitting within or around the tracks 64, and may be coupled to the reservoir, such as by adhesive, metal or elastic bands that encircle the followers 66 and reservoir 12, or other convenient coupling method. For example, the tracks 64 may be vertical grooves within at least a part of the stabilizer 16, through which the followers 66 move freely. The manner in which the followers 66 are in contact with each other prevents the reservoir 12 from tilting or toppling over.

With reference to FIG. 12, the stabilizer 16 may be one or more flexible retaining straps 68 that encircle the reservoir 12. The retaining straps 68 may be tied, coupled, affixed, or otherwise in contact with at least a portion of the stabilizer 16. For example, the stabilizer 16 may include a buckle or snap to which the retaining straps 68 are securely coupled.

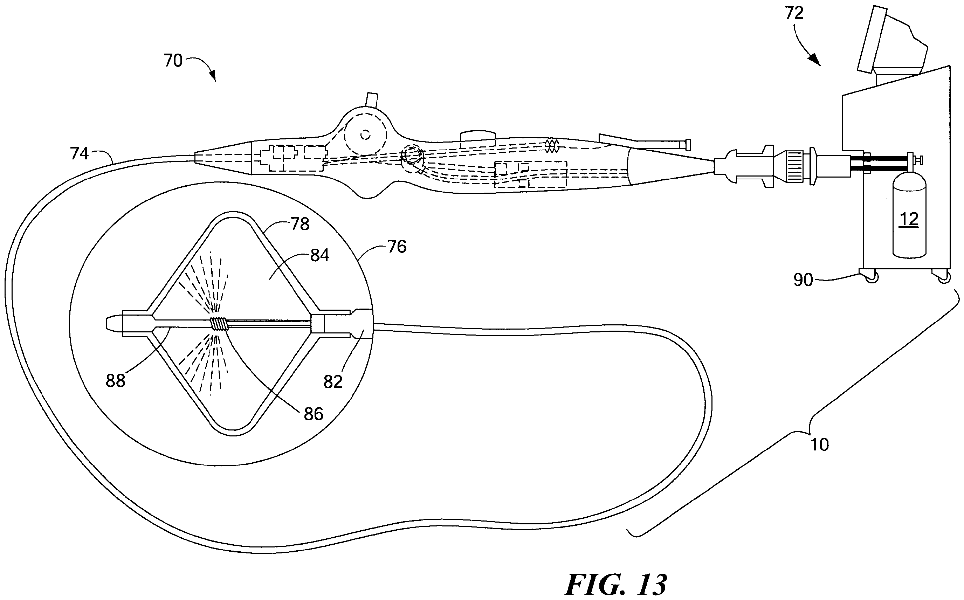

FIG. 13 illustrates the medical system 10, which includes the reservoir 12 and one or more of the stabilizers 16 as shown and described in FIGS. 1-12. The medical system 10, as shown in FIG. 13, may further include a medical device such as an ablation catheter 70 or any other cryogenic medical device in communication with a control unit 72. The ablation catheter 70 may generally include a flexible elongate body 74 having a distal end 76 that includes an ablation or treatment element 78 (for example, an expandable element as shown in FIG. 13), a handle 80, and one or more lumens 82 through which cryogenic fluid may be injected or removed from the distal end 76 or in which electrical components or a guide wire may be located. The ablation or treatment element 78 may include a fluid expansion chamber 84, a fluid injection element 86, and a shaft 88, in addition to other features included in various embodiments (for example, a second expandable element, one or more electrodes, etc.). The control unit 72 may house the reservoir 12 and may include a power source, conduits, connectors, and one or more displays, buttons, knobs, or other user input devices. Further, the control unit 72 may be movable (for example, the control unit 72 may be coupled to casters 90, as shown in FIG. 13)

The medical system 10 may thus be used for cryogenic medical treatment according to methods including measuring the weight of the reservoir 12 or other container of cryogenic fluid with a load sensor 14, and allowing the reservoir 12 a limited range of movement relative to the load sensor 14. Fluid communication may be between the reservoir 12 and a control unit 72, and restraining the reservoir 12 from tipping relative to the load sensor 14, which may thereby enhance accuracy in using the load sensor 14 to measure the weight of the reservoir 12.

It will be appreciated by persons skilled in the art that the present invention is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the system and devices disclosed herein are not necessarily exclusive of each other and may be included in a variety of different combinations or configurations without departing from the scope of the invention. A variety of modifications and variations are possible in light of the above teachings without departing from the scope of the invention, which is limited only by the following claims.

## Claims

1. A medical device (10), comprising:
a load sensor (14) coupled to a base;
a reservoir (12) containing cryogenic fluid and having a resting position on top of a surface of the load sensor (14);
a conduit (20) being in fluid communication with the reservoir (12), the conduit (20) acting as a stabilizer (16) restraining the reservoir (12) within a limited range of motion relative to the sensor (14), the conduit (20) comprising:
a first tube (36), the first tube (36) having a substantially constant length; and
a flexible second tube (38), the flexible second tube (38) being more flexible that the first tube (36);
an inflexible connector element (47) configured to facilitate connection of the first tube (36) of the conduit (20) to the reservoir (12); and
a coupling (40) including a bracket (42) having one or more guide members (44) coupled to the base at a location that is a distance from the load sensor (14) and the conduit (20) being attached to, affixed to or otherwise engaging the coupling (40) at the junction of the first tube (36) and the second tube (38).

2. The medical device (10) of claim 1, wherein the stabilizer (16) allows vertical movement of the reservoir (12) relative to the load sensor (14).

3. The medical (10) device of claim 1, wherein the stabilizer (16) is operable to resist tipping of the reservoir (12).

4. The medical device (10) of claim 1, wherein the stabilizer (16) is operable to substantially maintain a predetermined orientation of the reservoir (12) relative to the load sensor (14).

5. The medical device (10) of claim 1, wherein the inflexible connector element (47) is directly coupled with the reservoir (12).

6. The medical device (10) of claim 1, wherein a portion of the stabilizer (16) is positioned a vertical distance from the load sensor (14), defining a gap between the portion of the stabilizer (16) and the reservoir (12).

7. The medical device (10) of claim 1, further comprising a control unit (72) and the conduit (20) providing fluid communication between the reservoir (12) and the control unit (72), the stabilizer (16) resisting tipping of the reservoir (12) and disruption of the fluid communication.

8. The medical device (10) of claim 1, wherein the load sensor (16) is operable to measure the weight of the reservoir (12) and the cryogenic fluid.

## Patentansprüche

1. Medizinische Vorrichtung (10), die umfasst:
einen Lastsensor (14), der mit einer Basis gekoppelt ist;
einen Behälter (12), der Kryoflüssigkeit enthält und eine Ruheposition oben auf einer Oberfläche des Lastsensors (14) aufweist;
eine Leitung (20), die mit dem Behälter (12) in Flüssigkeitsverbindung steht, wobei die Leitung (20) als ein Stabilisator (16) wirkt, der den Behälter (12) innerhalb eines begrenzten Bewegungsbereichs relativ zu dem Sensor (14) zurückhält, wobei die Leitung (20) umfasst:
ein erstes Rohr (36), wobei das erste Rohr (36) eine im Wesentlichen konstante Länge aufweist; und
ein flexibles zweites Rohr (38), wobei das flexible zweite Rohr (38) flexibler als das erste Rohr (36) ist;
ein unflexibles Verbinderelement (47), das konfiguriert ist, um eine Verbindung des ersten Rohrs (36) der Leitung (20) mit dem Behälter (12) zu erleichtern; und
eine Kopplung (40), die einen Stützwinkel (42) beinhaltet, der eine oder mehrere Führungsglieder (44) aufweist, die an einer Stelle mit der Basis gekoppelt sind, die in einem Abstand von dem Lastsensor (14) liegt, und wobei die Leitung (20) an der Anschlussstelle des ersten Rohrs (36) und des zweiten Rohrs (38) an der Kopplung (40) befestigt ist, angebracht ist oder anderweitig mit dieser in Eingriff steht.

2. Medizinische Vorrichtung (10) nach Anspruch 1, wobei der Stabilisator (16) eine vertikale Bewegung des Behälters (12) relativ zu dem Lastsensor (14) ermöglicht.

3. Medizinische Vorrichtung (10) nach Anspruch 1, wobei der Stabilisator (16) betriebsfähig ist, um einem Kippen des Behälters (12) standzuhalten.

4. Medizinische Vorrichtung (10) nach Anspruch 1, wobei der Stabilisator (16) betriebsfähig ist, um eine zuvor bestimmte Ausrichtung des Behälters (12) relativ zu dem Lastsensor (14) im Wesentlichen aufrechtzuerhalten.

5. Medizinische Vorrichtung (10) nach Anspruch 1, wobei das unflexible Verbinderelement (47) mit dem Behälter (12) direkt gekoppelt ist.

6. Medizinische Vorrichtung (10) nach Anspruch 1, wobei ein Abschnitt des Stabilisators (16) in einem vertikalen Abstand von dem Lastsensor (14) positioniert ist, wobei ein Spalt zwischen dem Abschnitt des Stabilisators (16) und dem Behälter (12) definiert wird.

7. Medizinische Vorrichtung (10) nach Anspruch 1, die ferner eine Steuereinheit (72) umfasst und wobei die Leitung (20) eine Flüssigkeitsverbindung zwischen dem Behälter (12) und der Steuereinheit (72) bereitstellt, wobei der Stabilisator (16) dem Kippen des Behälters (12) und einer Unterbrechung der Flüssigkeitsverbindung standhält.

8. Medizinische Vorrichtung (10) nach Anspruch 1, wobei der Lastsensor (16) betriebsfähig ist, um das Gewicht des Behälters (12) und der Kryoflüssigkeit zu messen.

## Revendications

1. Dispositif médical (10) comprenant :
un capteur de charge (14) couplé à une base ;
un réservoir (12) contenant du fluide cryogénique et ayant une position de repos au-dessus d'une surface du capteur de charge (14) ;
un conduit (20) étant en communication fluidique avec le réservoir (12), le conduit (20) agissant comme un stabilisateur (16) retenant le réservoir (12) dans une plage de mouvement limitée par rapport au capteur (14), le conduit (20) comprenant :
un premier tube (36), le premier tube (36) ayant une longueur sensiblement constante ; et
un second tube flexible (38), le second tube flexible (38) étant plus flexible que le premier tube (36) ;
un élément de connecteur rigide (47) configuré pour faciliter la connexion du premier tube (36) du conduit (20) au réservoir (12) ; et
un raccord (40) comprenant un support (42) ayant un ou plusieurs éléments de guidage (44) couplés à la base au niveau d'un emplacement qui est à une certaine distance du capteur de charge (14) et le conduit (20) étant attaché, fixé ou autrement entrant en prise avec le raccord (40) au niveau de la jonction du premier tube (36) et du second tube (38).

2. Dispositif médical (10) selon la revendication 1, dans lequel le stabilisateur (16) permet un mouvement vertical du réservoir (12) par rapport au capteur de charge (14).

3. Dispositif médical (10) selon la revendication 1, dans lequel le stabilisateur (16) peut fonctionner pour résister au basculement du réservoir (12).

4. Dispositif médical (10) selon la revendication 1, dans lequel le stabilisateur (16) peut fonctionner pour maintenir sensiblement une orientation prédéterminée du réservoir (12) par rapport au capteur de charge (14).

5. Dispositif médical (10) selon la revendication 1, dans lequel l'élément de connecteur rigide (47) est directement couplé au réservoir (12).

6. Dispositif médical (10) selon la revendication 1, dans lequel une partie du stabilisateur (16) est positionnée à une distance verticale du capteur de charge (14), définissant un espace entre la partie du stabilisateur (16) et le réservoir (12).

7. Dispositif médical (10) selon la revendication 1, comprenant en outre une unité de commande (72) et le conduit (20) assurant une communication fluidique entre le réservoir (12) et l'unité de commande (72), le stabilisateur (16) résistant au basculement du réservoir (12) et à la perturbation de la communication fluidique.

8. Dispositif médical (10) selon la revendication 1, dans lequel le capteur de charge (16) peut fonctionner pour mesurer le poids du réservoir (12) et du fluide cryogénique.
